# FASCICULE DE BREVET EUROPEEN

(11) **EP 2 178 805 B1**
(45) Date de publication et mention de la délivrance du brevet: **27.03.2013**
(21) Numéro de dépôt: 08828762.8
(22) Date de dépôt: 08.07.2008
(51) Int. Cl.: C03C 1/00, C03C 3/078, C03C 3/097, C03C 3/112, C03C 4/00, C03C 8/08, C03C 12/00

(54) **VERRES BIOACTIFS DOPES EN STRONTIUM**
STRONTIUMDOTIERTE BIOAKTIVE GLÄSER
STRONTIUM DOPED BIOACTIVE GLASSES

(30) Priorité: 09.07.2007 FR 0704952
(43) Date de publication de la demande: 28.04.2010
(73) Titulaire: Centre National de la Recherche Scientifique, 75016 Paris (FR); Université Blaise Pascal - Clermont-Ferrand II, 63006 Clermont-Ferrand Cedex (FR); Université Paris Descartes, 75006 Paris (FR)
(72) Inventeur: JALLOT, Edouard, F-63360 Saint-Beauzire (FR); LAO, Jonathan, F-63000 Clermont-Ferrand (FR); NEDELEC, Jean-Marie, F-63190 Moissat (FR); SAUTIER, Jean-Michel, F-75019 Paris (FR); ISAAC, Juliane, F-75019 Paris (FR)
(74) Mandataire: Corizzi, Valérie
(86) Numéro de dépôt international: PCT/FR2008/000985
(87) Numéro de publication internationale: WO 2009/027594

(56) Documents cités:
- EP-A2- 0 251 695
- WO-A-2007/144662
- WO-A1-00/66086
- WO-A1-97/45070
- DE-A1- 4 020 893
- JP-A- 62 231 668
- US-A- 4 560 666
- US-A- 5 352 715
- US-A- 6 051 247
- US-B1- 6 756 060
- GALLIANO ET AL: "A study by density measurements and indentation tests of a calcium silicophosphate bioactive glass with different MgO or SrO contents" JOURNAL OF NON-CRYSTALLINE SOLIDS, NORTH-HOLLAND PHYSICS PUBLISHING. AMSTERDAM, NL, vol. 191, no. 3, 1 décembre 1995 (1995-12-01), pages 311-320, XP022270320 ISSN: 0022-3093

## Description

L'invention a pour objet de nouveaux verres bioactifs comprenant du, ou dopés en strontium, un procédé pour leur préparation et leur utilisation dans des procédés de réparation ou de reconstruction des os.

Les os sont constitués d'un réseau de fibres de collagène et de cristaux de phosphate de calcium hydratés et carbonatés. Des cellules appelées ostéocytes, qui comprennent les ostéoblastes et les ostéoclastes, sont insérées dans ce réseau. Elles sont alimentées par de très petits vaisseaux sanguins.

Lorsqu'un os est endommagé, les ostéoclastes éliminent les fragments abîmés et les ostéoblastes reconstruisent le réseau de collagène et favorisent la production d'enzymes qui vont permettre le dépôt d'apatite cristalline hydroxylée carbonatée, jusqu'à la réparation du défaut osseux.

Ce procédé naturel étant lent, il est habituel d'aider la réparation de l'os à l'aide de ciments osseux ou de prothèses plus ou moins importantes en fonction des dimensions de la zone endommagée. Une greffe osseuse est parfois nécessaire lorsque la reconstruction de l'os ne se fait pas ou est trop lente.

Dans tous les cas de réparation d'un défaut osseux, il est important, parallèlement à la mise en place d'une structure de remplacement, de favoriser la reconstruction du tissu osseux, qui va progressivement coloniser ou prendre la place du substitut osseux.

Dans le cas de certaines pathologies, et notamment l'ostéoporose, il est important de contrer la dégradation du tissu osseux en stimulant le travail des ostéoblastes.

Pour toutes ces applications, des verres bioactifs ont été développés depuis de nombreuses années. Les verres bioactifs réagissent chimiquement avec les fluides biologiques, le produit de la réaction est une hydroxyapatite qui favorise la formation de la matrice osseuse et la croissance osseuse.

Les premières céramiques bioactives ont été développées par L.L. Hench (L.L. Hench et al., J. Biomed. Mater. Res. 1971, 2, 117-141 ; L.L. Hench et al., J. Biomed. Mater. Res. 1973, 7, 25-42).

Les premiers verres bioactifs ont été préparés à partir de SiO₂ de P₂O₅ et de CaO et Na₂O. Les oxydes de silicium et de phosphore sont des formateurs de réseau qui participent à la cohésion du réseau vitreux. Les alcalins et alcalino terreux comme le sodium et le calcium ne présentent pas cette capacité et viennent modifier le réseau vitreux en y introduisant des ruptures de chaines qui sont à l'origine de la faible température de fusion de ces verres associée à un désordre structural accru. Leur présence a pour conséquence une plus grande réactivité des verres bioactifs dans un environnement aqueux. Cette réactivité permet la formation d'hydroxyapatite en milieu physiologique et favorise donc la reconstruction osseuse.

Le bioverre qui a été le plus étudié est un verre sodo-silico-phosphocalcique dit Bioglass® ou Bioverre de Hench. Sa composition de base est 55% SiO₂ - 20% CaO - 20% Na₂O - 5% P₂O₅. Les propriétés bioactives remarquables de ce matériau ne sont plus à démontrer. Le Bioglass® reste à l'heure actuelle un des matériau bioactifs (induisant une réponse spécifique des cellules) les plus intéressants.

De nombreux développements ont été fait dans le domaine des verres bioactifs depuis leur découverte (M. Vallet-Regi et al., Eur. J. Inorg. Chem. 2003, 1029-1042), tels que l'incorporation de différents atomes ou l'incorporation de principes actifs. Les compositions des verres bioactifs ont été optimisées de façon à favoriser la prolifération des ostéoblastes et la formation de tissus osseux (WO 02/04606). L'incorporation d'argent a été proposée notamment pour conférer des propriétés antibactériennes aux verres bioactifs (WO 00/76486).

Toutefois, l'incorporation d'un nouvel élément dans un verre bioactif ne va pas sans présenter des difficultés : en effet, tout atome introduit dans une composition de verre bioactif a une influence sur le comportement de ce verre et sur ses propriétés, en particulier sur la façon dont ce verre relargue les éléments dont il est composé. Et le verre bioactif doit à la fois bien se dissoudre pour permettre la formation d'hydroxyapatite, mais la vitesse de dissolution doit être contrôlée pour permettre une colonisation progressive de l'hydroxyapatite et un relargage prolongé d'éventuels actifs.

Enfin, les conditions de production de verres bioactifs doivent être adaptées à chaque nouvelle composition.

Les propriétés de bioactivité des verres et leur vitesse de dissolution sont influencées par leur composition et leur texture. La composition de base d'un verre bioactif est de la forme SiO₂-CaO-P₂O₅ ou SiO₂-Na₂O-CaO-P₂O₅. Cependant, le rôle de certains éléments traces durant les différentes étapes dans le processus de dissolution, de relargage d'ions et les réactions physico-chimiques conduisant à la bioactivité a été très peu étudié.

Le strontium est naturellement présent dans les tissus osseux et il peut être incorporé dans les apatites lors des phases de croissance des précipités (formation d'apatites déficientes en calcium). Par ailleurs, la littérature décrit cet élément comme pouvant jouer un rôle sur les réactions cellulaires. Le strontium améliore les propriétés mécaniques de l'os et il a une influence sur la solubilité des apatites. Il est aussi impliqué dans l'ostéoporose puisqu'il améliore les propriétés mécaniques des hydroxyapatites. Il permet, *in vivo* d'avoir un meilleur lien avec les tissus environnants. Bien qu'améliorant l'adhésion cellulaire, il diminue légèrement la croissance des ostéoblastes en culture et augmente la production de lactate deshydrogénase. Le strontium permet aussi d'immobiliser les cellules et l'adhérence des cellules pourrait être meilleure quand le biomatériau est dopé en Sr (E. Canalis et al., Bone 1996, 18, 517-523 ; G. Boivin et al., J. Bone Miner. Res. 1996, 11, 1302-1311 ; P. Marie et M. Hott, Metabolism 1986, 35, 547-551 ; P. Marie, Current Opinion in Pharmacology 2005, 5, 633-636).

Un objet de l'invention a été la mise au point d'un nouveau matériau bioactif qui présente des propriétés améliorées par rapport aux matériaux de l'art antérieur.

Le matériau de l'invention est une composition de verre bioactif comprenant du strontium. Selon une première variante de l'invention, ce verre bioactif est issu d'un procédé sol-gel. Cette composition se caractérise par la présence des éléments ci-dessous dans les proportions indiquées :

| | |
|---|---|
| SiO₂ : | de 40 à 75% |
| CaO : | de 15 à 30% |
| SrO : | de 0,1 à 10% |
| P₂O₅ : | de 0 à 10% |
| Na₂O : | de 0 à 20% |
| MgO : | de 0 à 10% |
| ZnO: | de 0 à 10% |
| CaF₂ : | de 0 à 5% |
| B₂O₃ : | de 0 à 10% |
| Ag₂O : | de 0 à 10% |
| Al₂O₃ : | de 0 à 3 % |
| MnO : | de 0 à 10% |
| Autres : | de 0 à 10% |

Les pourcentages sont des pourcentages en masse par rapport à la masse totale de la composition.

Avantageusement, la somme des masses des éléments SiO₂, CaO, SrO, P₂O₅ représente 98 à 100%, encore mieux 99 à 100 %, et préférentiellement 99,9 à 100 % de la masse totale de la composition du matériau de l'invention.

De façon avantageuse le matériau de l'invention est constitué de :

| | |
|---|---|
| SiO₂ : | de 45 à 75% |
| CaO : | de 15 à 30% |
| SrO : | de 2 à 8% |
| P₂O₅ : | de 0 à 10% |
| Autres éléments : | de 0 à 1 %, préférentiellement de 0 à 0,5%, |

en masse par rapport à la masse totale de la composition.

Les matériaux de l'invention peuvent être issus d'un procédé sol-gel et être sous forme d'une poudre libre ou d'une poudre compactée, sous forme de fibres ou encore sous forme d'un revêtement sur un support ou d'un monolithe ou d'un verre fritté.

Selon une variante de l'invention, les matériaux peuvent être issus d'un procédé de fusion à haute température suivie d'une trempe. Dans ce cas ils sont définis par la composition suivante :

| | |
|---|---|
| SiO₂ : | de 45 à 55% |
| Na₂O : | de 10 à 25% |
| CaO : | de 10 à 25% |
| SrO : | de 0,1 % à 10% |
| P₂O₅ : | de 0 à 10% |
| MgO : | de 0 à 10% |
| ZnO: | de 0 à 10% |
| CaF₂ : | de 0 à 5 % |
| B₂O₃ : | de 0 à 10% |
| Ag₂O : | de 0 à 10% |
| Al₂O₃ : | de 0 à 3 % |
| MnO : | de 0 à 10% |
| Autres : | de 0 à 10%. |

Les pourcentages sont des pourcentages en masse par rapport à la masse totale du matériau.

Avantageusement, la somme des masses des éléments SiO₂, Na₂O, CaO, SrO, P₂O₅ représente 98 à 100%, encore mieux 99 à 100 %, et préférentiellement 99,9 à 100 % de la masse totale de la composition du matériau de l'invention.

De façon avantageuse, selon cette variante, le matériau de l'invention est constitué de :

| | |
|---|---|
| SiO₂ : | de 45 à 55% |
| Na₂O : | de 15 à 25% |
| CaO : | de 15 à 25% |
| SrO : | de 2 à 8% |
| P₂O₅ : | de 0 à 10% |
| Autres éléments : | de 0 à 1%, préférentiellement de 0 à 0,5%, |

en masse par rapport à la masse totale de la composition.

Les matériaux de l'invention peuvent être issus d'un procédé de fusion et être sous forme de monolithe ou de verre fritté.

L'expression « verre bioactif » désigne un matériau du type verre inorganique dont l'oxyde de silicium est le composant majoritaire et qui est capable de se lier à des tissus vivants lorsqu'il est placé dans un fluide physiologique.

Les verres bioactifs sont bien connus de l'homme du métier et sont décrits notamment dans « An introduction to Bioceramics », L. Hench et J. Wilson, World Scientific Edition, New Jersey (1993).

Les matériaux de l'invention sont biocompatibles ce qui signifie que lorsqu'ils sont mis en contact avec un organisme vivant, et notamment avec un organisme humain ou animal, ils n'induisent pas de réaction des systèmes de protection de l'organisme tels que le système immunitaire notamment. Le terme biocompatible implique également que lorsque le matériau est implanté chez un patient, il ne produit pas d'effets cytotoxiques ou de réactions systémiques.

Les matériaux de l'invention sont à la fois biocompatibles et bioactifs. Par rapport aux matériaux de l'art antérieur, ils possèdent l'avantage de renforcer les propriétés mécaniques de l'os et de favoriser la liaison entre l'hydroxyapatite et les tissus environnants. Les biomatériaux de l'invention ont donc des propriétés qui les rendent supérieurs aux biomatériaux de l'art antérieur dans la réparation des défauts osseux et dans la prévention et/ou le traitement des déficiences osseuses de toutes origines.

Les matériaux de l'invention peuvent être préparés par un procédé sol-gel, qui présente de nombreux avantages : températures de production plus faibles par rapport aux autres procédés, matériaux plus homogènes, contrôle aisé de la composition finale et contrôle de la porosité et de la surface spécifique du matériau. La bioactivité étant conditionnée par la structure du matériau en plus de sa composition chimique, on a constaté que les matériaux issus d'un procédé sol-gel étaient particulièrement intéressants dans la mesure où il est facile par ce procédé de contrôler leur vitesse de dissolution ainsi que la vitesse de relargage du strontium.

Les matériaux de l'invention peuvent être préparés par un procédé qui comporte les étapes de mélange en solution des alcoxydes métalliques, hydrolyse, formation du gel et chauffage de façon à produire une matrice poreuse ou un verre dense.

Le procédé sol-gel est mis en oeuvre avec une composition de matière telle que celle décrite ci-dessus à 3 composants ou plus, dont au moins SiO₂, CaO, SrO, et éventuellement P₂O₅ et/ou d'autres oxydes.

Dans une première étape les précurseurs des composants, le solvant (eau et éventuellement un alcool tel que l'éthanol) sont mélangés en présence d'un catalyseur, acide ou basique.

De façon plus détaillée un tétraalcoxysilane tel que le tétraéthoxysilane est utilisé comme précurseur de SiO₂, un trialkylphosphate tel que le triéthyl phosphate est employé comme précurseur de P₂O₅, le nitrate de calcium tétrahydraté ou un autre sel de calcium (chlorure, acétate, fluorure, oxalate,...) est employé comme précurseur de CaO et le nitrate de strontium ou un autre sel de strontium (chlorure, acétate, fluorure, oxalate,...) comme précurseur de SrO.

Les réactions d'hydrolyse et de condensation sont catalysées par le même catalyseur, comme par exemple HCl. La structure du gel qui se forme est conditionnée notamment par la valeur du pH de la solution dans laquelle se font ces réactions. Au seuil de percolation le réseau tridimensionnel formé s'étend à travers tout le milieu réactionnel et l'on obtient un gel.

Vieillissement : cette étape implique de maintenir le gel immergé dans le solvant pendant plusieurs heures à plusieurs jours. Pendant le vieillissement les polycondensations se poursuivent jusqu'à ce que toutes les espèces réactives aient réagi. Cette étape, dite de synérese, contribue à diminuer la porosité et à renforcer le gel. La durée et la température du vieillissement permettent de contrôler la porosité du gel.

Pendant l'étape du séchage, le liquide présent dans les pores en est expulsé. Des contraintes capillaires se développent et provoquent le craquement du gel, à moins d'opérer dans des conditions qui réduisent les tensions solide-liquide interfaciales, comme par exemple par addition de tensioactifs.

La stabilisation et la densification peuvent être obtenus par des moyens thermiques ou chimiques, dans des conditions qui permettent d'éliminer les groupes silanol de surface.

De préférence on opère par chauffage de façon à dégrader les autres éléments présents dans le gel tels que les nitrates. De préférence le chauffage se fait à une température supérieure ou égale à 600°C.

Le produit final est alors obtenu sous forme d'une poudre. La taille des pores est comprise entre 1 nm et 50 µm. De préférence, on obtient une poudre avec des pores de 2 à 50 nm de diamètre.

Les utilisations qui peuvent être faites des matériaux de l'invention sont les suivantes : comblement de défauts osseux, recouvrement d'implants métalliques, stimulation de la croissance osseuse dans les cas de dégénérescence osseuse.

Ces applications peuvent être mises en oeuvre de différentes façons :
Les matériaux de l'invention peuvent être introduits localement par chirurgie ou par injection : dans une zone où un défaut osseux a été constaté, par exemple par radiographie. Il est possible de combler un défaut osseux par l'insertion d'un matériau de l'invention sous forme de poudre libre ou de poudre compactée.

La poudre obtenue par le procédé peut être utilisée en l'état, par exemple en chirurgie osseuse ou maxillodentaire, en comblement de défauts osseux. Elle peut être injectée sous forme d'une composition thérapeutique dans les zones où une stimulation de la croissance osseuse est attendue. Cette poudre peut être compactée sous forme de pastilles à l'aide d'une presse, de façon à former un objet tridimensionnel qui est utilisé en chirurgie.

Selon une variante de l'invention, des fibres de verre bioactif peuvent être préparées en passant par un procédé sol-gel, en mettant en oeuvre les étapes suivantes : le sol est extrudé à travers une filière. Les fibres obtenues sont mises à vieillir, séchées et stabilisées thermiquement. Les fibres peuvent ensuite être tissées ou agglomérées à l'aide d'un liant, comme par exemple une solution de carboxyméthylcellulose. Un réseau de fibres agglomérées peut ensuite être utilisé pour produire un verre fritté par chauffage dans un four à des températures produisant la dégradation du liant.

Les fibres de bioverre de l'invention peuvent être utilisées en l'état, comme fil de suture ou sous forme de tissu, en chirurgie. Elles peuvent être employées dans des compositions incluant d'autres matériaux.

Les matériaux de l'invention peuvent être utilisés seuls ou en combinaison avec d'autres moyens favorisant la réparation et/ou la régénération du tissu osseux. Des compositions thérapeutiques, notamment des compositions destinées à l'injection ou à une administration par chirurgie, et comprenant au moins un matériau de l'invention, constituent un autre objet de l'invention. Ces compositions peuvent comprendre tout support pharmaceutiquement acceptable pour l'utilisation qui est faite de la composition : notamment support d'injection.

Outre les verres bioactifs de l'invention, on peut prévoir que des formulations à injecter ou à mettre en place par chirurgie comprennent également un ou plusieurs composés choisis parmi les antibiotiques, les antiviraux, les agents cicatrisants, les anti-inflammatoires, les immunosuppresseurs, les facteurs de croissance, les anticoagulants, les agents vascularisants, les analgésiques, un plasmide...,

Les matériaux de l'invention peuvent aussi être déposés sur un élément métallique ou céramique tel qu'une vis, une plaque, un tube, une valve qui est implanté dans l'organisme comme prothèse.

Les matériaux de l'invention peuvent être associés à une matrice, telle qu'une matrice osseuse destinée à être greffée. L'association des matériaux de l'invention au greffon, notamment lorsque celui-ci est allogène, favorise son incorporation dans l'organisme.

Des prothèses recouvertes d'un matériau de l'invention peuvent être fabriquées de façon connue en trempant une prothèse classique, en métal ou en céramique, ou un greffon osseux débarrassé de son réseau cellulaire, ou un polymère biocompatible, dans une solution sol-gel ou par projection par plasma de la composition sur la prothèse, puis en poursuivant par un chauffage à une température supérieure à 600°C qui provoque la formation du verre bioactif.

Une prothèse, métallique ou en céramique ou en polymère, ou une matrice osseuse, recouvertes partiellement ou sur la totalité de leur surface par un matériau de l'invention constituent un autre objet de l'invention.

Les matériaux de l'invention peuvent également être préparés par voie sol gel sous forme de monolithes de forme contrôlée. Selon cette variante, le procédé comporte un contrôle de l'étape de séchage et de densification de façon à éviter les fissures dans le gel. Le sol est mis à gélifier à 60 °C dans un récipient en PTFE dont la forme définit la forme finale du monolithe.

De tels monolithes sont employés en chirurgie, par exemple pour combler un défaut osseux.

Les matériaux de l'invention peuvent également être introduits par chirurgie ou par injection dans une localisation connue pour sa fragilité osseuse comme le col du fémur chez les individus souffrant d'ostéoporose.

Les matériaux de l'invention peuvent également être introduits autour des articulations pour favoriser la réparation et/ou la régénération du cartilage lorsque celui-ci est endommagé.

Les matériaux et les compositions de l'invention peuvent être utilisés pour la réparation d'un cartilage, soit à la suite d'une blessure ayant entraîné une dégradation du cartilage, soit dans le cadre d'un traitement de l'ostéoarthrose. Les maladies inflammatoires des articulations d'une façon générale peuvent constituer des situations où l'utilisation d'un matériau selon l'invention peut être bénéfique.

Les matériaux de l'invention peuvent également être préparés par un procédé de fusion en mélangeant les oxydes métalliques et les autres composants, en les portant à la fusion puis en les refroidissant. La température de fusion est déterminée en grande partie par le choix des composants du verre. Elle se situe entre 900 et 1500°C environ. Les matériaux obtenus dans ce cas sont monolithiques et non poreux.

Selon cette variante, on peut également préparer un verre fritté, de façon connue à partir de la composition de verre fondu qui est frittée de façon à produire un matériau particulaire.

Dans le cas des matériaux obtenus sous forme de monolithes (fusion ou sol-gel), ces matériaux peuvent être utilisés en chirurgie, par insertion dans un site à traiter, soit qu'un défaut osseux nécessite d'être comblé soit qu'un relargage d'apatite strontiée y soit utile pour renforcer la structure osseuse existante.

Un autre objet de l'invention consiste en des solutions obtenues à partir des verres bioactifs, par dissolution des verres bioactifs dans un milieu aqueux. Ces solutions peuvent être produites en plaçant le verre bioactif de l'invention dans une solution aqueuse puis en laissant le verre se dissoudre et en filtrant le milieu. La solution filtrée est récupérée. Elle favorise la croissance des ostéoblastes. Elle peut être utilisée dans une composition, notamment une composition injectable, pour administration dans une zone localisée de l'organisme où il est souhaitable de stimuler la croissance des ostéoblastes. Elle peut aussi être utilisée en laboratoire pour cultiver des cellules. Elle peut être utilisée pour préparer un médicament de toute forme telle que solide, semi-solide, liquide, comme par exemple sous forme de comprimés, de pastilles, de poudre, de solution liquide, de suspension, de suppositoires.

Les matériaux, compositions et prothèses de l'invention sont particulièrement utiles pour réparer les défauts osseux et/ou cartilagineux et les carences associés aux pathologies et aux blessures dans les cas suivants : formation de tissus osseux dans une fracture, réparation de défauts osseux tels que ceux dus à l'ablation d'une tumeur ou d'un kyste, le traitement d'anomalies dentaires ou du squelette, reconstruction dentaire et périodontique, notamment remplacement de l'os alvéolaire, dans les pathologies périodontiques qui entraînent une perte osseuse, ou pour remplir une cavité entre la dent et la gencive ou pour remplacer provisoirement une dent extraite, dans les cas d'ostéoporose.

On choisit une forme adaptée à l'usage qui doit en être fait et le plus souvent une forme permettant l'injection ou l'insertion par voie chirurgicale sur le site où une déficience osseuse doit être traitée.

Un autre objet de l'invention consiste en l'utilisation d'un matériau tel que décrit ci-dessus pour la fabrication d'une prothèse ou d'un médicament destiné à prévenir ou traiter l'une ou l'autre des pathologies qui ont été décrites ci-dessus.

Les biomatériaux élaborés sont des verres bioactifs nanostructurés. Les études physico-chimiques réalisées lors des interactions biocéramiques/milieu biologique mettent en évidence des propriétés de bioactivité permettant *in fine* la formation d'une couche phosphocalcique à la surface des matériaux. Cette couche permet dans le cas des matériaux bioactifs un lien intime avec les tissus osseux. De plus, le contrôle de la texture (porosité) et de la composition en éléments majeurs et traces (Sr) permettent de moduler les propriétés de dissolution et de bioactivité de ces matériaux. Ainsi, les verres élaborés relarguent du strontium dans des concentrations physiologiques. Ce relargage contrôlé d'un élément trace (présent dans l'os) peut induire une réponse spécifique des cellules.

Différentes compositions de matériaux conformes à la présente invention ont été produites et l'on a étudié leur comportement en solution. On a constaté qu'à l'interface entre la composition et le milieu dans lequel elle est immergée il se forme une hydroxyapatite dont la vitesse de formation peut être contrôlée. On a aussi constaté le relargage de strontium sous forme ionique dans le milieu et son incorporation dans la couche phosphocalcique produite *in situ.*

Le strontium, comme le calcium, est un modificateur de réseau dans les compositions de l'invention. Tous deux ont des rayons ioniques proches. Et on a néanmoins constaté que la présence de strontium jouait un rôle dans la vitesse de relargage des constituants de la composition alors que le calcium a peu d'influence sur ce paramètre.

On a notamment constaté que l'augmentation de la quantité de strontium dans la composition entraînait une diminution de la vitesse de relargage du strontium, de calcium, phosphore, du silicium.

Ainsi, non seulement les compositions de l'invention permettent de relarguer du strontium dans leur environnement lorsqu'elles sont placées dans un liquide physiologique, mais elles permettent aussi de le faire de façon contrôlée.

En résumé, les compositions de l'invention permettent :
- le relargage dans des concentrations physiologiques du strontium directement sur le site d'implantation,
- l'amélioration de la minéralisation osseuse,
- le contrôle de la dissolution et du relargage des matériaux,
- la possibilité d'implanter et d'injecter le matériau sur le site choisi,
- la formation d'une couche phosphocalcique à la périphérie des matériaux en milieu biologique.

### PARTIE EXPERIMENTALE

### I - Protocole de synthèse

Les verres bioactifs ont été élaborés sous forme de poudres. Le Tableau I-1 décrit les précurseurs chimiques fournis par Sigma-Aldrich (USA).

**Tableau I-1 : caractéristiques des précurseurs chimiques.**

| | Formule | Masse molaire (g.mol⁻¹) | Pureté (%) |
|---|---|---|---|
| Tétraéthoxysilane (TEOS) | C₈H₂₀O₄Si | 208,33 | 99,999 |
| Triéthylphosphate (TEP) | C₆H₁₅O₄P | 182,16 | 99,8 |
| Nitrate de calcium tétrahydraté | Ca(NO₃)₂-4H₂O | 236,15 | 99,99 |
| Nitrate de strontium | Sr(NO₃)₂ | 211,63 | 99 |

Pour effectuer la réaction d'hydrolyse du TEOS, nous avons utilisé un cosolvant (éthanol EtOH). Nous avons employé comme catalyseur de l'acide chlorhydrique HCl.

Concernant le protocole de synthèse, l'eau distillée nécessaire à l'hydrolyse est tout d'abord mélangée à l'acide chlorhydrique HCl (2N) et à l'éthanol EtOH (99%), qui permet outre d'obtenir une solution homogène après introduction du TEOS, de bien dissoudre les cristaux de Ca(NO₃)₂-4H₂O. Les proportions d'eau, d'éthanol et d'acide chlorhydrique sont détaillées dans le tableau 1-2.

**Tableau I-2 : proportions d'eau, d'éthanol et d'acide chlorhydrique.**

| | |
|---|---|
| H₂O/(TEOS + TEP) | R ₘₒₗₐᵢᵣₑ = 12 |
| H₂O/HCl | R _{volumique} = 6 |
| H₂O/EtOH | R _{volumique} = 1 |

Ces réactifs sont mélangés dans un ballon sous agitation magnétique pendant 15 minutes. Le TEOS est alors ajouté au mélange et, après 30 minutes, le TEP y est versé ainsi qu'un volume égal d'éthanol. Au bout de 20 minutes, les cristaux de Ca(NO₃)₂-4H₂O sont introduits. Le mélange est alors agité pendant 60 minutes supplémentaires.

Puis la solution est placée dans un verre de montre et séchée dans une étuve à 60°C pour gélification. Cette opération dure 4 heures, et le sol en ressort totalement gélifié. L'étuve est ensuite portée à 125°C pendant 24 heures. Le gel est à cet instant complètement fragmenté et est broyé finement au mortier pour la dernière étape de la synthèse : la calcination à 700°C pendant 24 heures, qui outre la densification va permettre d'évaporer intégralement les résidus d'alcool et de nitrate piégés dans les pores. Le produit final est obtenu sous forme d'une fine poudre blanche.

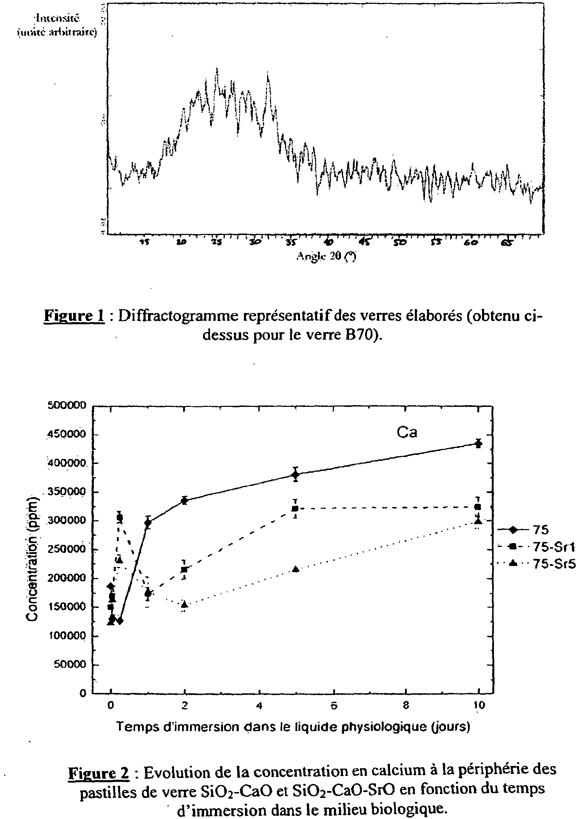

La figure 1 représente le diffractogramme sur un verre, caractérisé par la méthode d'analyse cristalline de diffraction des rayons X. Les diffractogrammes obtenus pour les autres verres sont semblables à celui-ci. L'absence de pics de diffraction indique que les verres élaborés sont bien amorphes.

### II- Caractéristiques des verres élaborés

### II-1- Composition

L'étude de la composition des verres élaborés a été réalisée par spectrométrie d'émission atomique (ICP-AES). Les résultats de l'analyse par spectrométrie d'émission atomique sont détaillés dans les tableaux II-1 et II-2. Les verres élaborés ont des concentrations en oxydes conformes aux valeurs escomptées.

**Tableau II-1 : concentrations en oxydes mesurées en ICP-AES pour les verres binaires et ternaires (% massique).**

| | B75 | B72,5 | B70 | B67,5 |
|---|---|---|---|---|
| SiO₂ Théorique | 75 | 72,5 | 70 | 67,50 |
| SiO₂ Expérimentale | 72,20 | 71,49 | 68,25 | 63,75 |
| | | | | |
| P₂O₅ Théorique | - | 2,5 | 5 | 7,5 |
| P₂O₅ Expérimentale | - | 2,48 | 4,85 | 6,95 |
| | | | | |
| CaO Théorique | 25 | 25 | 25 | 25 |
| CaO Expérimentale | 24,50 | 24,36 | 25,76 | 24,13 |

**Tableau II-2: concentrations en oxydes mesurées en ICP-AES pour les verres dopés en strontium (% massique).**

| | B75-Sr1 | B75-Sr5 | B67,5-Sr1 | B67,5-Sr5 |
|---|---|---|---|---|
| SiO₂ Théorique | 75 | 75 | 67,5 | 67,5 |
| SiO₂ Expérimentale | 74,22 | 74,08 | 67,16 | 64,93 |
| | | | | |
| P₂O₅ Théorique | - | - | 7,5 | 7,5 |
| P₂O₅ Expérimentale | - | - | 7,04 | 7,62 |
| | | | | |
| CaO Théorique | 24 | 20 | 24 | 20 |
| CaO Expérimentale | 23,60 | 19,03 | 23,31 | 20,25 |
| | | | | |
| SrO Théorique | 1 | 5 | 1 | 5 |
| SrO Expérimentale | 0,83 | 3,83 | 0,81 | 4,27 |

### II-2- Texture

Les surfaces spécifiques des verres sont été mesurées par adsorption de gaz sur un appareil Autosorb Quantachrome fonctionnant à 77.4 K par la méthode BET. L'adsorbat utilisé est de l'azote de haute pureté (99.999%) avec une section efficace d'adsorption de la molécule d'azote de 0.162 nm² pour le calcul de la surface spécifique. Avant mesure les échantillons sont dégazés sous vide (p< 1Pa) à 120°C pendant 12 h. Les surfaces spécifiques sont calculées à partir des masses des échantillons dégazés.

Un minimum de 5 points ont été utilisés pour la mesure des quantités de gaz adsorbées dans un domaine de pression partielle p/p₀ comprise entre 0.05 et 0.3 (avec P₀ : pression de vapeur saturante).

Les surfaces spécifiques sont comprises entre 50 et 150 m²/g. La taille moyenne des pores est comprise entre 1 nm et 101 nm.

### III - Étude in vitro de la bioactivité

Il est clairement établi que la faculté d'un biomatériau à se lier aux tissus vivants est subordonnée à sa capacité à former une couche apatitique au contact de fluides biologiques imitant le plasma sanguin. Les essais *in vitro* sont donc un outil puissant pour évaluer la bioactivité d'un matériau.

Le milieu biologique dans lequel les verres bioactifs ont été immergés est du DMEM (Dulbecco's Modified Eagle Medium). La composition du DMEM est similaire à celle du plasma sanguin chez l'homme (Tableau III-1). Le pH du DMEM à 37°C vaut 7,43, une valeur analogue à celle du plasma.

**Tableau III-1: concentrations ioniques du plasma sanguin chez l'homme (mmol.L⁻¹).**

| Na⁺ | K⁺ | Mg²⁺ | Ca²⁺ | Cl⁻ | CO₃²⁻ | PO₄³⁻ | SO₄²⁻ |
|---|---|---|---|---|---|---|---|
| 142,0 | 5,0 | 1,5 | 2,5 | 147,8 | 4,2 | 1,0 | 0,5 |

### III-1- Protocole expérimental

Les échantillons de verres bioactifs ont été étudiés sous forme de poudre et sous forme de pastilles : disques de 13 mm de diamètre et 2 mm de hauteur, obtenues en compactant 90 mg de poudre au moyen d'une presse. Les verres bioactifs sont susceptibles d'être utilisés dans des applications cliniques sous ces deux formes il est donc intéressant d'étudier ces deux types d'échantillons. La bioactivité s'y opère à des échelles différentes de temps et de dimensions.

### III-1.1- Les échantillons sous forme de pastilles

Les échantillons de pastilles ont été immergés dans 45 mL de DMEM durant les délais suivants : 1 heure, 6 heures, 1 jour, 2 jours, 5 jours, 10 jours.

Après immersion, les pastilles sont récupérées puis séchées sous atmosphère ambiante, tandis qu'une partie du DMEM est prélevée afin d'être dosée en ICP-AES. Les échantillons de pastilles destinés à être caractérisés par la microsonde ionique sont inclus dans de la résine. Des sections transverses du matériau sont ensuite réalisées à l'aide d'un microtome Leica RM 2145. Les coupes, de 30 µm d'épaisseur, sont effectuées perpendiculairement à la surface du disque. Finalement, les coupes sont placées sur des supports en Mylar percés en leur centre d'un trou de 3 mm de diamètre. C'est la zone de l'échantillon placée au-dessus du trou qui est sondée par le microfaisceau d'ions.

### III-1.2- Les échantillons sous forme de poudre

N'étant pas massifs comme les échantillons de pastilles et ayant en outre une structure poreuse, les échantillons de grains de poudre réagissent plus vite que les échantillons de pastille. Notre étude sur les poudres s'est focalisée sur la caractérisation de 4 verres bioactifs : les verres B75, B67,5, B75-Sr5 et B67,S-Sr5. Pour chaque verre, 10 mg de poudre ont été immergés dans du DMEM suivant un rapport [surface spécifique]/[volume de DMEM] fixé à 500 cm⁻¹, ceci afin d'évaluer l'impact sur la bioactivité de la composition du verre et non de sa surface de contact avec le milieu biologique. Les temps d'immersion dans le DMEM sont les suivants : 1 heure, 6 heures, 1 jour, 2 jours, 3 jours, 4 jours.

### III-2- Caractérisation des réactions physico-chimiques lors des interactions verre bioactif/milieu biologique

Afin de mieux comprendre les réactions physico-chimiques conduisant à la formation de différentes couches en périphérie des bioverres, il est primordial d'analyser localement la distribution des éléments à l'interface matériaux/fluides biologiques. Ces analyses nécessitent l'utilisation de méthodologies ayant une bonne sensibilité et une excellente résolution spatiale. Dans ce cadre, nous avons réalisé des cartographies chimiques de l'interface à l'échelle micrométrique par la méthode PIXE (Particles Induced X-ray Emission). Cette méthode est basée sur la fluorescence X induite par faisceau d'ions (généralement des protons). Elle permet de réaliser des cartographies multiéléments simultanées et des mesures de concentrations sur les éléments majeurs, mineurs et traces (ppm) avec une résolution spatiale de l'ordre du micron.

### III-2.1- Imagerie chimique multiélémentaire à la périphérie des pastilles de verre dopées au strontium après immersion dans le milieu biologique

Les cartographies multiélémentaires ont été enregistrées lors de l'analyse par microfaisceau d'ions des pastilles de verre binaires et ternaires dopées au strontium. Des cartographies multiélémentaires ont été obtenues pour chacun des verres avant interaction et après 1 heure, 6 heures, 1 jour, 2 jours, 5 jours et 10 jours d'interaction avec le milieu biologique. La discussion est faite en effectuant une comparaison avec l'étude des pastilles de verre binaires et ternaires non dopées.

Grâce aux images chimiques obtenues, il a été possible de réaliser le suivi de la distribution en silicium, calcium, phosphore, strontium et magnésium à l'interface verre bioactif/milieu biologique en fonction du temps d'interaction entre le matériau et le liquide. Les mesures de concentrations dans les verres réalisées en PIXE fournissent alors des informations locales sur la réactivité du matériau afin de disposer d'informations sur la réactivité globale des échantillons, l'évolution des concentrations dans le milieu biologique a été suivie par des mesures en ICP-AES. La confrontation de ces résultats est donc nécessaire et donnera des informations complémentaires sur la réactivité des matériaux. Concernant l'analyse PIXE, les échantillons de pastille ont été sondés au moyen d'un faisceau de protons de diamètre 2 µm et d'intensité 500 pA. Les cartographies ont été accomplies par le balayage de zones carrées de côté compris entre 40 µm et 400 µm suivant les régions d'intérêt.

Les cartographies des verres de composition SiO₂-CaO-SrO révèlent que l'ajout de strontium dans la composition du verre diminue la dissolution du matériau comparativement à un verre SiO₂-CaO. Cet effet est visible sur les cartographies du calcium.

Concernant la distribution du strontium, cet élément est réparti uniformément jusqu'à 1 heure d'interaction. Une partie du strontium semble ensuite être relarguée de la périphérie du matériau et le strontium est détecté dans des proportions plus importantes dans les régions intérieures des pastilles.

Le dopage en strontium s'avère également avoir une incidence sur le développement de la couche phosphocalcique. Ainsi, alors que la présence de phosphore était détectée à la périphérie du verre B75 dès 1 heure d'interaction, cet élément n'est décelé qu'après 6 heures d'interaction pour les verres B75-Sr1 et B75-Sr5. De même, les traces de magnésium ne sont décelées qu'au bout de 6 heures pour les verres SiO₂-CaO-SrO contre 1 heure pour le verre SiO₂-CaO. Par la suite, la couche Ca-P-Mg croît de manière similaire au verre binaire. Après 10 jours d'interaction, on observe trois régions dans ces pastilles de verre. Les régions les plus intérieures de la pastille sont constituées du réseau vitreux originel. La couche périphérique est une région étendue riche en calcium et en phosphore, dans laquelle sont présentes des traces de magnésium et de strontium. Enfin, entre ces deux régions, on constate la présence d'une zone intermédiaire localement enrichie en calcium.

Les cartographies multiélémentaires concernant les verres de composition SiO₂-CaO-P₂O₅-SrO attestent également d'un ralentissement de la dissolution du matériau en comparaison du verre non dopé B67,5. L'ajout de strontium se traduit ainsi par le ralentissement du relargage du calcium. La capacité de ces matériaux à former une couche Ca-P-Mg-Sr est néanmoins avérée après quelques jours d'interaction.

### III-2.2- Mesures locales des concentrations élémentaires lors des interactions pastilles de verre/milieu biologique

Suivant la répartition des espèces chimiques, les cartographies multiélémentaires ont été sectorisées en différentes régions d'intérêt. Chaque fois que la région périphérique riche en calcium et en phosphore a été repérée, des masques de mesures ont été définis, permettant d'y calculer les concentrations élémentaires. Suivant la région d'intérêt, des masques de 5 à 20 µm de côté ont été définis, l'épaisseur de la couche Ca-P augmentant avec le temps d'immersion. En appliquant cette méthodologie, l'évolution des concentrations des espèces Ca, P, Si, Sr et Mg à la périphérie et au centre des verres a pu être suivie. Pour un temps d'interaction et un verre donnés, les valeurs de concentration portées sur les graphes sont la moyenne des concentrations relevées dans plusieurs régions d'intérêt.

### Evolution des concentrations à la périphérie des pastilles de verre

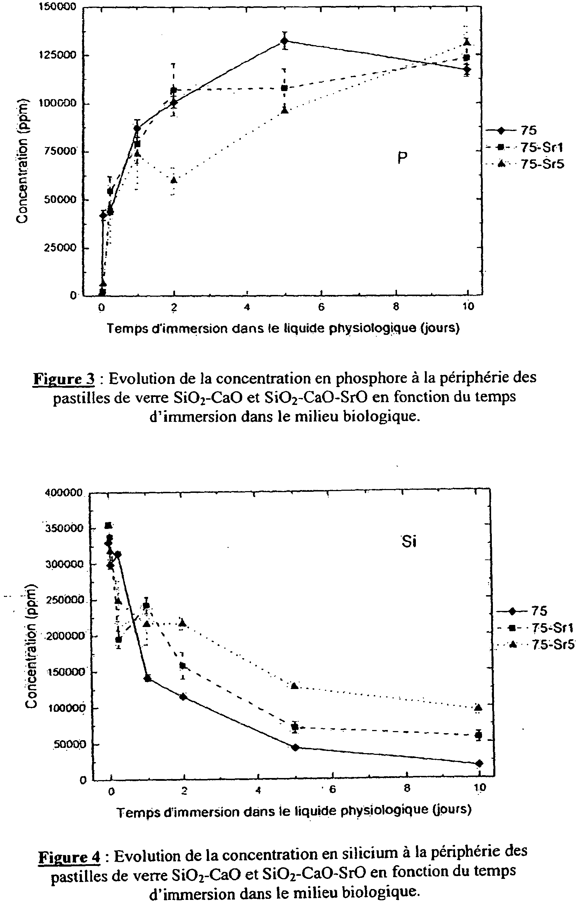

Les figures 2, 3, 4 et 5 montrent l'évolution des concentrations élémentaires à la périphérie des verres SiO₂-CaO-SrO. Les concentrations mesurées à la périphérie du verre B75 (SiO₂-CaO) y sont également portées afin d'opérer une comparaison. La figure 2 représentant l'évolution des concentrations en calcium, indique que les verres B75-Sr1 et B75-Sr5 adoptent un comportement résolument différent du verre B75. Pour les verres SiO₂-CaO-SrO la concentration en calcium commence par augmenter lors des premières heures d'interaction avec le liquide physiologique. L'augmentation relative de la concentration en calcium est due à ce que dans le même temps la concentration en silicium diminue fortement (figure 4). Ceci tend à indiquer que chez les verres dopés en strontium, le relargage du calcium n'avance pas aussi rapidement que la décomposition du réseau de silicium : le relargage du calcium est ralenti et semble affecter une quantité plus limitée de cations de la matrice. Ce n'est qu'au-delà de 6 heures d'interaction que la concentration en calcium chute jusqu'à un minimum atteint après 1 jour d'interaction pour le verre B75-Sr1 et après 2 jours pour le verre B75-Sr5. Le minima atteint est plus élevé que celui du verre B75 : l'ampleur de la dissolution est donc moindre chez les matériaux dopés en strontium.

Après l'étape de relargage, la quantité de calcium présente en périphérie des verres croît, mais cette augmentation est moins rapide pour les verres SiO₂-CaO-SrO que pour le verre SiO₂-CaO. Après 10 jours d'immersion, la proportion de calcium contenue dans la couche Ca-P périphérique des verres dopés en strontium est proche de 30% massiques, ce qui est inférieur à la quantité de calcium détecté dans la couche Ca-P périphérique du verre B75 (44% massique). Mais il faut tenir compte du fait que les matrices des verres B75-Sr1 et B75-Sr5 contiennent initialement moins de calcium.

La figure 4 montre que la décroissance de la concentration en silicium à la périphérie des matériaux est d'autant plus lente que la proportion en strontium dans la matrice vitreuse originelle est élevée. Après 10 jours d'interaction, la couche périphérique du verre B75-Sr1 est encore composée de 6% de silicium, celle du verre B75-Sr5 de 9% de silicium.

Concernant le phosphore (figure 3) la tendance observée semble commune aux trois verres B75, B75-Sr1 et B75-Sr5 ; à savoir, une augmentation rapide de la concentration de cet élément en périphérie des pastilles. Un extremum est finalement atteint après 10 jours d'interaction. La teneur en phosphore de la périphérie des verres B75, B75-Sr1 et B75-Sr5 est alors voisine de 12%.

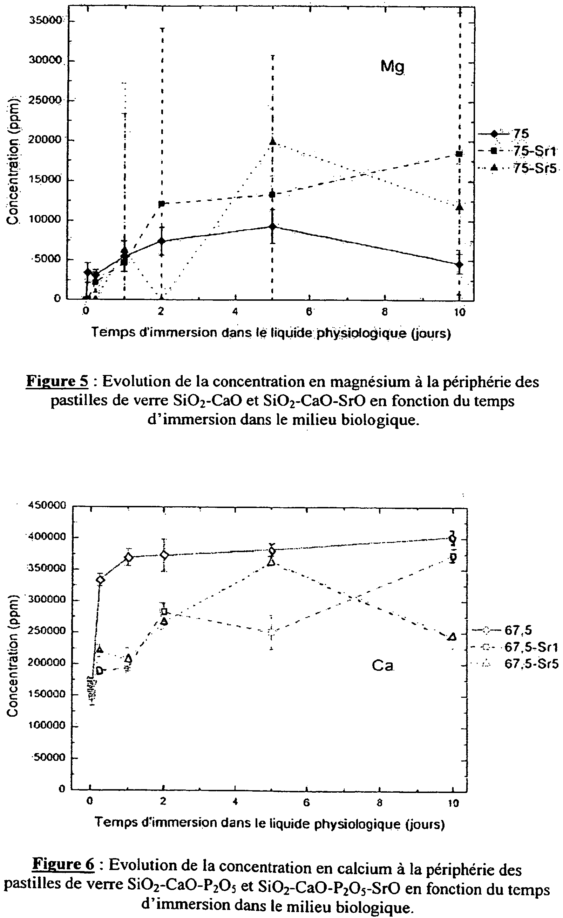

Des traces de magnésium sont détectées dans la couche qui se développe à la surface des verres SiO₂-CaO-SrO (figure 5). La proportion de magnésium augmente à mesure que le temps d'immersion se prolonge et donc que la couche périphérique s'étend à la surface des verres. La quantité de magnésium incorporée à la périphérie des pastilles s'avère être plus importante pour les verres SiO₂-CaO-SrO que pour le verre binaire SiO₂-CaO.

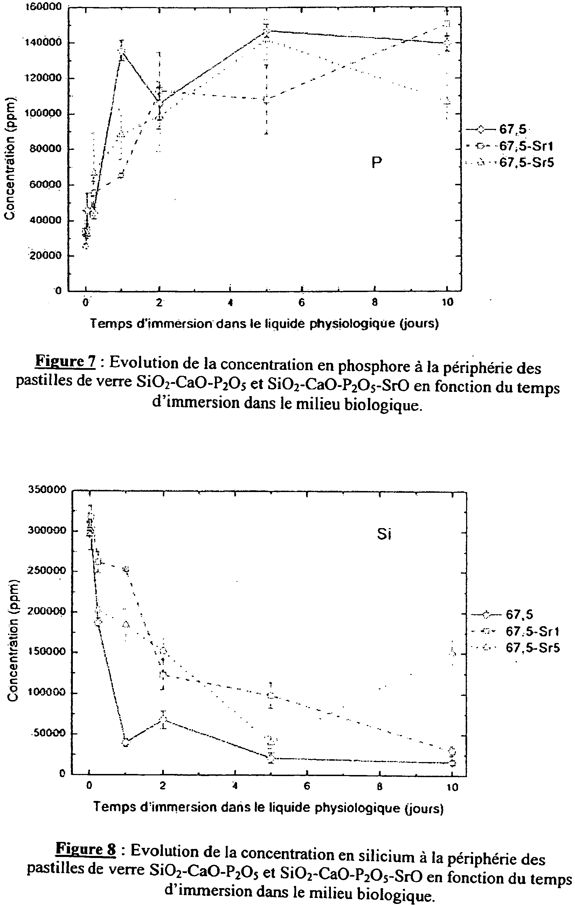

Les figures 6, 7, 8 et 9 montrent l'évolution des concentrations élémentaires à la périphérie des verres SiO₂-CaO-P₂O₅-SrO. Sur la figure 6, on peut observer que l'évolution des concentrations en calcium à la périphérie des verres B67,5-Sr1 et B67,5-Sr5 augmente de manière analogue à celle du verre B67,5 ; cependant l'évolution est plus lente et le calcium est présent en quantité moins importante pour les verres dopés en strontium. Sur la figure 8, on remarque que la cinétique de décroissance de la concentration en silicium est moins rapide pour les verres contenant du strontium que pour le verre ternaire B67,5. Après 10 jours d'interaction, de plus fortes concentrations de silicium subsistent à la périphérie des verres SiO₂-CaO-P₂O₅-SrO. Ces observations sont le signe que chez les verres dopés en strontium, la décomposition du réseau vitreux est opérée sur une profondeur moindre.

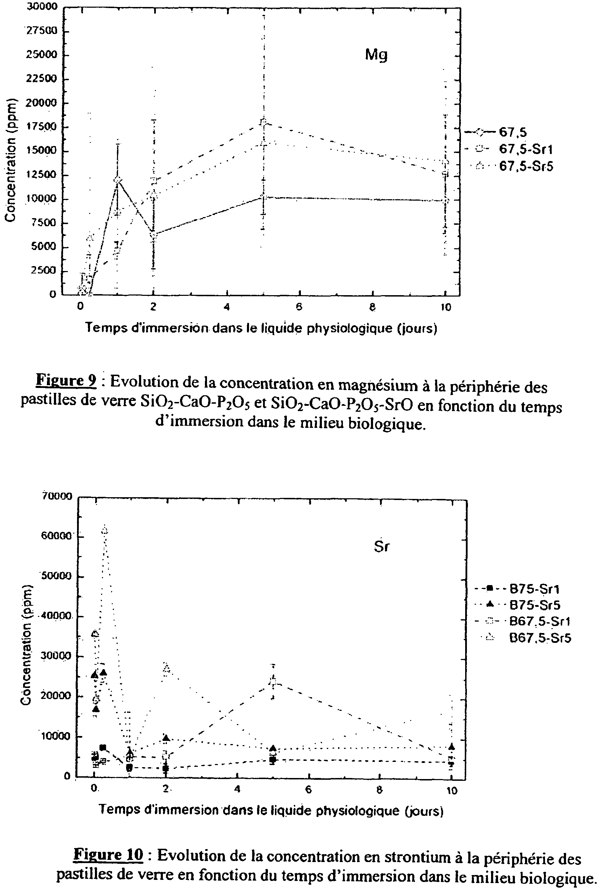

La quantité de phosphore décelée dans la région périphérique des pastilles augmente rapidement avec le temps d'immersion (figure 7). L'évolution des concentrations est commune aux trois verres et la couche périphérique est à terme constituée de 11 à 15% de phosphore. Concernant le magnésium, cet élément est au bout de 10 jours présent à hauteur du pourcent à la périphérie des pastilles. On note sur la figure 9 qu'une plus grande quantité de magnésium est incorporée chez les verres composés de strontium B67,5-Sr1 et B67,5-Sr5 comparativement au verre B67,5.

La figure 10 représente l'évolution des concentrations en strontium à la périphérie des pastilles de verre SiO₂-CaO-SrO et SiO₂-CaO-P₂O₅-SrO. Sous l'effet des échanges ioniques et des réactions physico-chimiques ayant lieu en surface, d'importantes fluctuations dans la concentration du strontium sont observées, Il est néanmoins possible de dégager pour la couche périphérique une tendance générale à un léger appauvrissement de la quantité de strontium. Après 10 jours d'interaction, la périphérie des matériaux est d'autant plus riche en strontium que la proportion de cet élément était importante dans la matrice vitreuse originelle, et les concentrations mesurées sont inférieures aux valeurs avant interaction.

### Evolution des concentrations dans la région intérieure des pastilles de verre

Les mesures des concentrations élémentaires dans les régions intérieures des pastilles de verre, non directement exposées aux fluides biologiques, ont été faites pour les éléments Si, Ca, P, Sr et Mg. Comme remarqué précédemment, les phénomènes de diffusion et de migration des ions vers la périphérie du matériau entraînent des fluctuations dans la composition de la matrice vitreuse. Les principales variations sont observées pour les concentrations en silicium, calcium et strontium lors des 2 premiers jours d'interaction avec le milieu biologique. L'évolution de la concentration en phosphore démontre également une légère tendance à la hausse. Après 10 jours d'interaction, on observe que les concentrations des différents éléments constitutifs retournent à une valeur proche de celle qui était leur dans la matrice vitreuse originelle. Les régions intérieures des pastilles de verres dopés en strontium ont subi moins de modifications que les verres non dopés. L'amplitude et la cinétique de dissolution étant moindre chez les verres dopés, la couche Ca-P-Mg développée en périphérie ne semble pas s'étendre jusqu'aux régions les plus intérieures des pastilles de verre.

### Evolution des rapports atomiques à l'interface pastilles de verre/milieu biologique

On a étudié l'évolution des rapports atomiques Ca/P, Ca/Mg et Ca/Sr à l'interface verre/milieu biologique.

Lors des premières heures d'interaction, le rapport atomique Ca/P est plus élevé pour les verres SiO₂-CaO-SrO que pour le verre SiO₂-CaO. Cela tient au fait que le calcium, relargué en quantité moins importante pour les verres SiO₂-CaO-SrO est donc présent en plus fortes proportions à la surface de ces matériaux. Au-delà de 6 heures d'interaction, la dissolution et le relargage du calcium s'accélèrent pour les verres B75-Sr1 et B75-Sr5 ceci, ajouté à l'incorporation rapide de phosphore issu du milieu, résulte en la chute brutale du rapport Ca/P observée à 1 jour d'interaction. Puis, à mesure que le temps d'immersion dans le fluide biologique augmente, le rapport Ca/P tend vers une valeur limite proche de 1,7, qui est celle de l'hydroxyapatite stoechiométrique. Ainsi, après 10 jours d'immersion, on constate que la valeur du rapport Ca/P finalement atteinte est égale à 1,8 pour les verres B75-Sr1 et B75-Sr5, ce qui est plus proche de la valeur nominale de l'apatite stoechiométrique si l'on compare au résultat de 2,1 obtenu pour le verre B75. Concernant les verres SiO₂-CaO-P₂O₅-SrO : les rapports Ca/P mesurés à l'interface sont constamment inférieurs à ceux du verre SiO₂-CaO-P₂O₅. Ceci est du d'une part à la plus faible croissance de la concentration en calcium, et d'autre part à la plus faible proportion de calcium initialement présente dans ces matériaux respectivement 24% et 20% pour les verres B67,5-Sr1 et B67,5-Sr5, contre 25% pour B67,5. Après 10 jours d'interaction, la remarque émise pour les verres SiO₂-CaO-SrO est également valable pour les verres SiO₂-CaO-P₂O₅-SrO à savoir que le rapport Ca/P pour les verres dopés en strontium est plus proche de celui de l'hydroxyapatite stoechiométrique comparativement aux verres non dopés. Au bout de 10 jours d'immersion, ce rapport Ca/P vaut 1,6 pour le verre B67,5-Sr1 et 1,7 pour le verré B67,5-Sr5, contre 1,9 pour le verre B67,5.

### Evolution de la composition du milieu biologique lors des interactions avec les pastilles de verre

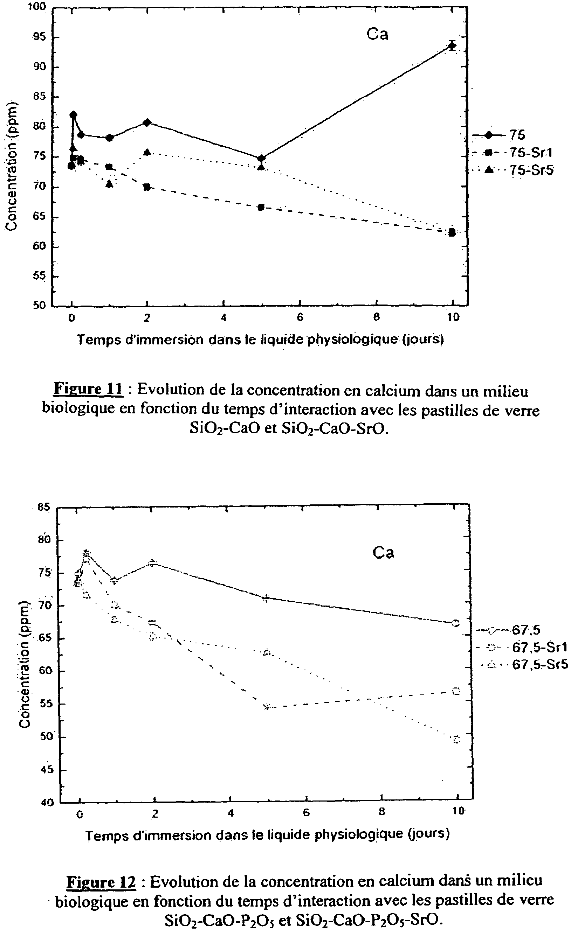

L'évolution de la concentration en calcium présente dans le DMEM (figures 11 et 12) présente de faibles variations lors des premières heures d'interaction. La quantité de calcium relarguée dans le milieu lors de la phase de désalcalinisation de la surface est plus faible pour les verres dopés en strontium que pour les verres B75 et B67,5. Ensuite, à mesure que le temps d'immersion s'allonge, on constate pour tous les verres dopés une diminution progressive de la concentration en calcium dans le milieu biologique. Le verre binaire B75 relargue d'importantes quantités de calcium si bien que cet élément était présent en plus forte concentration après 10 jours d'interaction qu'avant interaction ; cette observation n'est pas constatée pour les verres B75-Sr1 et B75-Sr5 Les verres dopés en strontium s'avèrent incorporer de plus grandes quantités de calcium comparées à celles relarguées. Ainsi, après 10 jours d'interaction, la concentration en calcium dans le milieu biologique n'est plus que de 62 ppm pour les verres B75-Sr1 et B75-Sr5 alors qu'elle était de 94 ppm pour le verre B75. Pour les verres B67,5-Sr1 et B67,5-Sr5, elle est respectivement égale à 5 et 49 ppm, alors qu'elle valait 67 ppm pour le verre B67,5.

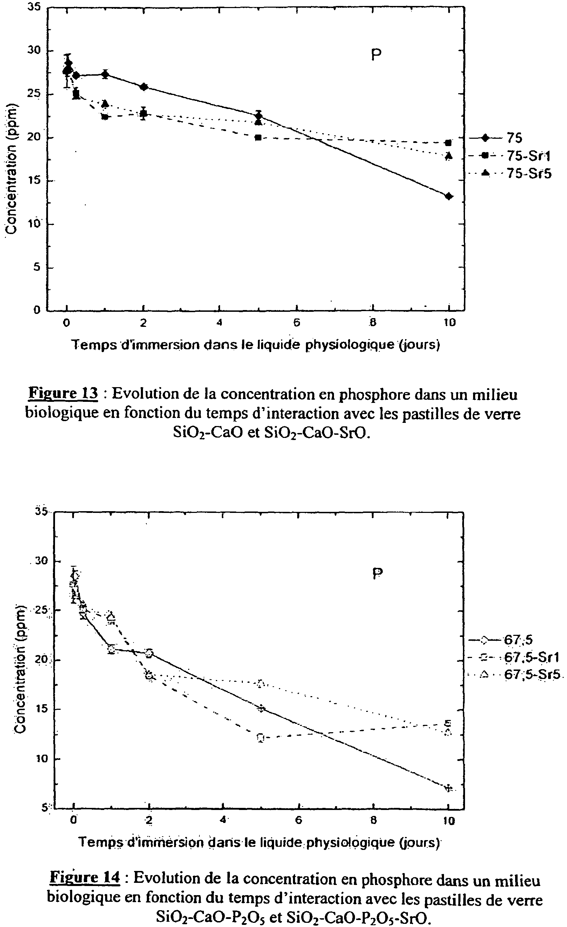

Les figures 13 et 14 représentent l'évolution de la concentration en phosphore présente dans le milieu biologique. Pour tous les verres, on constate une importante diminution de la concentration de cet élément au fil du temps. Les décroissances observées pour chacun des échantillons sont analogues. On note toutefois qu'après 5 jours d'interaction la consommation de phosphore semble ralentie chez les verres dopés en strontium. Ce pourrait être le signe que le système verre-milieu biologique parvient à un équilibre.

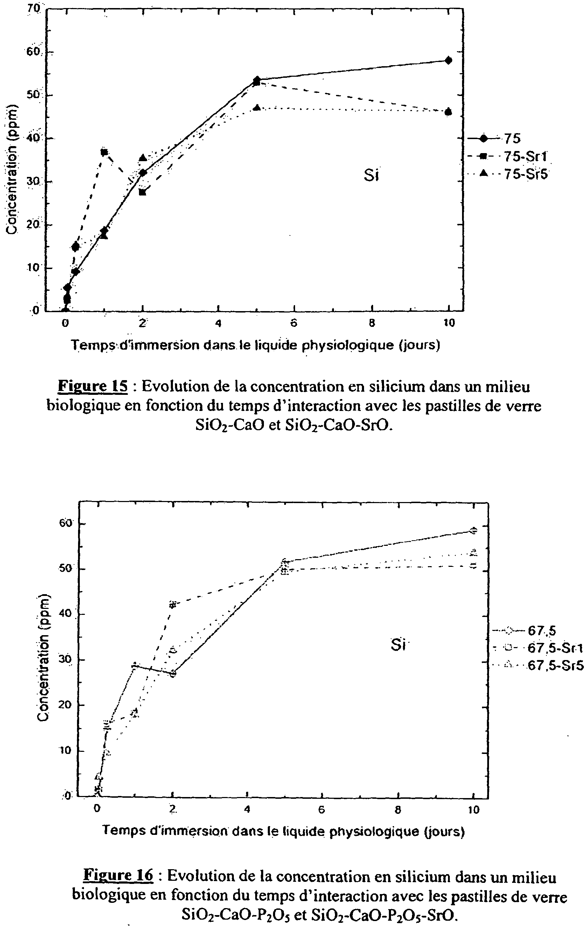

Concernant le silicium, les figures 15 et 16 montrent une tendance commune à tous les verres. A mesure que les réactions de dissolution décomposent le réseau vitreux sur des profondeurs de plus en plus larges, des concentrations de silicium de plus en plus élevées sont décelées dans le milieu biologique. Au bout de 10 jours d'interaction, les quantités de silicium relarguées dans le milieu biologique sont plus faibles pour les verres dopés en strontium. Ceci est un autre indice de la moindre amplitude de dissolution chez les verres dopés.

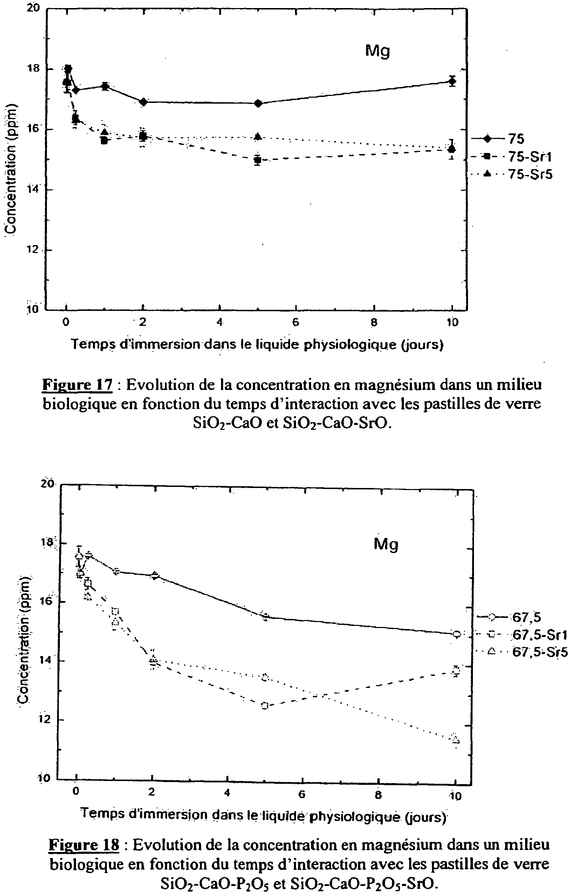

D'autre part, les figures 17 et 18 montrent que les verres dopés en strontium incorporent plus de magnésium que les autres verres. La concentration de cet élément décroît lentement avec le temps d'immersion et au bout de 10 jours, la diminution en magnésium est de 2 ppm pour les verres B75-Sr1 et B75-Sr5, 3 ppm pour le verre B675-Sr1 et 5 ppm pour le verre B67,5-Sr5.

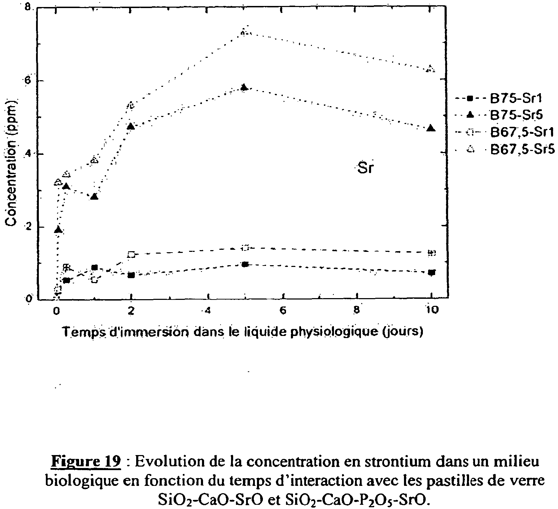

Enfin, les mesures de la concentration en strontium présente dans le milieu biologique complètent cette étude (figure 19). Initialement égale à zéro, la quantité de strontium dans le liquide physiologique s'élève à quelques ppm après le relargage de cet élément hors de la surface des verres. On remarque que les verres B75-Sr5 et B67,5-Sr5 relarguent 5 fois plus de strontium que les verres B75-Sr1 et B67,5-Srl, ce qui concorde avec les teneurs respectives en strontium de ces matériaux.

### III-2.4- Mesures locales des concentrations élémentaires lors des interactions grains de verre/milieu biologique

### Evolution des concentrations à la périphérie des grains de verre

L'analyse locale de la périphérie des grains révèle que les phénomènes observés pour les poudres sont la reproduction de ceux observés pour les pastilles, à des échelles de temps et de dimensions réduites toutefois. Les concentrations élémentaires affichent des tendances analogues à celles observées précédemment.

Ces observations s'appliquent également à l'évolution de la concentration en phosphore. Comme pour les pastilles, la concentration de cet élément augmente rapidement à la périphérie des grains. Après 4 jours d'interaction, le phosphore y est contenu à hauteur de 9-10% pour les verres dopés en strontium, et à hauteur de 16% pour le verre ternaire. Pour le verre B75, la quantité de phosphore croît promptement jusqu'à 6 heures d'interaction. Au-delà, la concentration en phosphore s'affaisse jusqu'à des valeurs quasi nulles. La couche phosphocalcique formée en bordure des grains de verre B75 apparaît donc instable et est rapidement dissoute sous l'action des fluides biologiques.

On constate que la concentration en silicium en bordure des grains de verre B75 décroît lors des premiers temps d'interaction, correspondant à la brisure du réseau vitreux sur la périphérie du matériau. Cependant, au-delà de 6 heures d'interaction, la couche phosphocalcique concentrique est dissoute et conséquemment les grains ne sont plus constitués que d'un noyau vitreux riche en silicium. Pour les verres B67,5, B75-Sr5 et B67,5-Sr5 le phénomène observé est différent : le réseau de silicium est progressivement décomposé sur les régions périphériques des grains, et par suite la concentration de cet élément diminue. On note que la décroissance est plus lente pour les verres dopés en strontium comparativement aux verres non dopés ; c'était aussi le cas pour les échantillons sous forme de pastille.

### IV- Evaluation préliminaire du comportement des cellules ostéoblastiques cultivées au contact du bioverre dopé en strontium (B75Sr5)

### IV-I- Etude in vitro - Méthode de culture

Les bioverres dopés en strontium B75Sr5 sont étudiés sous forme de granules. Préalablement à leur utilisation, les bioverres sont pesés et stérilisés à 180°C pendant 2 heures. Une préincubation des granules dans le milieu de culture (voir composition ci-dessous) est ensuite effectuée pendant 48 heures sous agitation. Suite à cette préincubation, les granules de bioverres sont immédiatement mis au contact des cellules.

Des cellules ostéoblastiques sont isolées par digestion enzymatique à partir de calvaria de foetus de rats âgés de 21 jours. Les calvaria sont disséquées en conditions stériles et les fragments sont incubés en présence de collagénase (Life technologies®) pendant 2 heures à 37°C. Les cellules dissociées des fragments osseux sont alors ensemencées dans des boîtes de culture (5ml) à une densité de 2.10⁵ cellules/ml. Lorsque la culture atteint le stade de subconfluence (environ 80% de la surface colonisée), les granules de bioverres sont ajoutés aux tapis cellulaires (20 mg/boîte de culture). Le milieu de culture est composé de DMEM (Invitrogen®), d'acide ascorbique (50 µg/mL), de 10 mM de β-glycérophosphate (Sigma®), de 50 UI/mL de Pénicilline-Streptomycine (Gibco®) et de 10% de sérum de veau foetal (SVF) (Hyclone®). Les cellules sont cultivées pendant 14 jours, dans un incubateur à 37°C dans une atmosphère humide à 5% de CO₂

### IV-2- Etude de l'interface grain de verre B75Sr5/cellules osseuses par Microscopie photonique à contraste de phase.

Les observations par microscopie photonique à contraste de phase permettent de suivre l'évolution, la maturation ainsi que la formation de nodules osseux autour et au contact du bioverre.

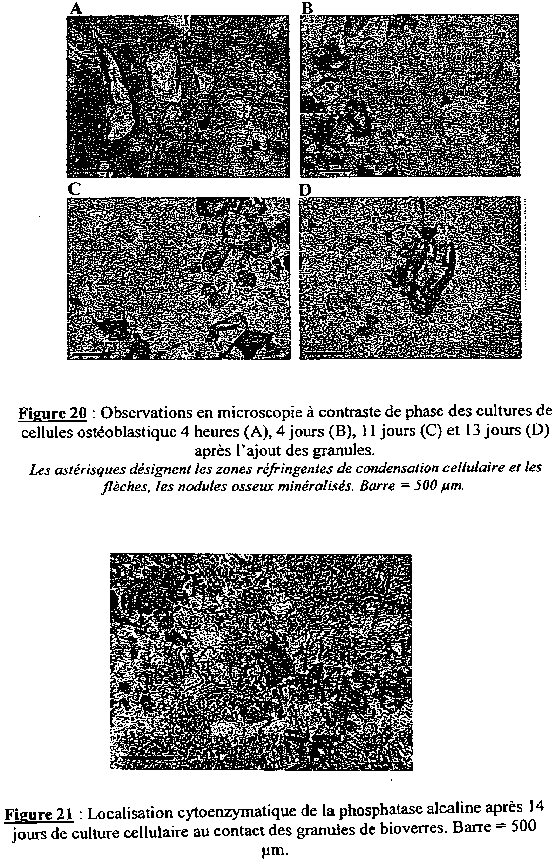

Durant les premiers jours de culture, les cellules prolifèrent (figure 20) et atteignent le stade de confluence entre le 3^{e} et le 4^{e} jour de culture (figure 20), immobilisant les granules dans le tapis cellulaire. Dans les jours suivants, les cellules continuent de proliférer et s'organisent en multicouches à la périphérie des granules. Cette organisation tridimensionnelle est visible dès le début de la deuxième semaine de culture sous forme de zones réfringentes (figure 20). A la fin de la deuxième semaine de culture, ces zones réfringentes sont très abondantes autour des granules et commencent à se propager sur l'ensemble du tapis cellulaire et dès le 13^{e} jour, nous observons l'apparition des premiers nodules osseux minéralisés (figure 20).

Ces résultats démontrent qu'en présence de granules de bioverres dopés en strontium, les cellules de calvaria de rats prolifèrent et se différencient en ostéoblastes actifs qui forment des nodules osseux minéralisés.

### N-3- Localisation cytoenzymatique de la phosphatase alcaline

Les cellules sont cultivées pendant 14 jours au contact des granules de bioverre. Ces cellules sont ensuite fixées dans une solution de fixation (mélange de citrate et d'acétone) à température ambiante pendant 30 secondes. Les échantillons cellulaires sont ensuite rincés et incubés dans une solution colorant les cellules synthétisant la phosphatase alcaline (solution de « fast blue salt RR » et naphtol phosphate, Sigma®) à température ambiante pendant 30 minutes à l'abri de la lumière. Après la réaction cytoenzymatique, les échantillons sont rincés puis examinés par microscopie photonique à contraste de phase.

Au 14^{e} jour de culture un marquage positif de la phosphatase alcaline, marqueur de la différenciation ostéoblastique, est observé au niveau de cellules situées autour et au contact des granules de bioverres (figure 21). Ces résultats indiquent que la présence de bioverres de type B75Sr5 permet une différenciation des cellules de calvaria de rats.

### IV-5- Etude en microscopie optique et microscopie électronique à transmission

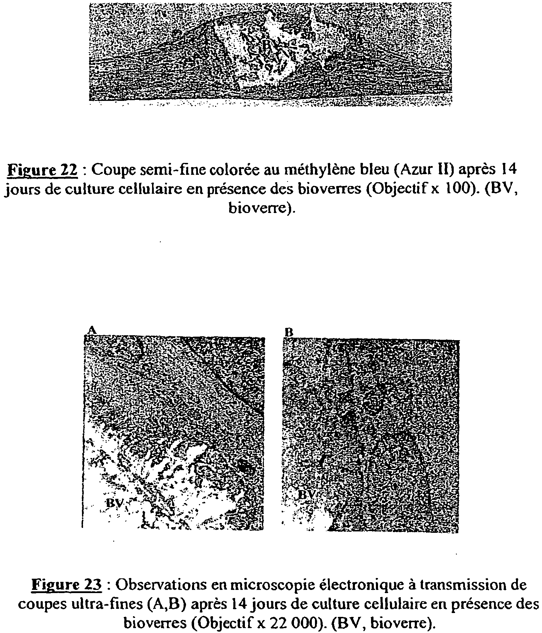

Les cellules sont traitées pour la microscopie électronique à transmission après 14 jours de culture au contact des granules de bioverres. Les cellules sont fixées dans une solution de Karnovsky (4% paraformaldehyde et 1% glutaraldehyde) puis les échantillons sont déshydratés par des bains d'éthanol croissants. Le tapis cellulaire avec les granules immobilisés sont ensuite inclus dans de l'Epon-Araldite, et des coupes semi-fines (figure 22) puis ultra-fines (figure 23) sont réalisées avec un couteau en diamant, perpendiculairement au tapis cellulaire. Les coupes ultra-fines sont collectées sur des grilles de cuivre puis colorées avec de l'acétate d'uranyle et du citrate de plomb. Les sections sont ensuite examinées avec un microscope électronique à transmission (Philips CM-12).

Nous observons sur les coupes semi-fines une organisation tridimensionnelle en multicouches des cellules autour des granules (figure 22).

Les observations en microscopie électronique à transmission révèlent la présence de nombreuses cellules au contact des granules (figure 23). Ces cellules ont des organites intracytoplasmiques développés, indiquant une forte activité cellulaire. Elles sont entourées d'une dense matrice extracellulaire riche en fibres de collagène. Nous pouvons également observer dans la matrice de multiples foyers de minéralisation. Enfin, un contact intime est observé entre la matrice, les cellules et la périphérie des granules.

La présence des bioverres n'altère pas les capacités de synthèses matricielles, puisque les cellules présentent tous les signes d'activité de synthèse (réticulum endoplasmique, mitochondries, noyau volumineux ....). Nous observons également la présence d'une matrice extracellulaire composée de nombreuses fibres de collagène.

### Conclusion sur l'étude biologique

L'ensemble de ces résultats démontre le caractère non cytotoxique des granules de bioverres dopés en strontium sur les cellules primaires issues de calvaria de rat. En effet, après 14 jours de culture au contact des bioverres, aucun signe de souffrance cellulaire n'est relevé et les cellules cultivées au contact de ces granules prolifèrent, s'organisent en structure tridimensionnelle et sont capables de synthétiser une matrice extracellulaire. De plus, l'activité phosphatase alcaline de ces cellules et l'apparition de nodules osseux minéralisés après 2 semaines de cultures indiquent que la présence des granules de bioverres ne nuit pas à la différenciation ostéoblastique des cellules étudiées.

## Revendications

1. Verre bioactif, **caractérisé en ce qu'**il est issu d'un procédé sol-gel et que sa composition se **caractérise par** la présence des éléments ci-dessous dans les proportions indiquées :
| | |
|---|---|
| SiO₂ : | de 40 à 75% |
| CaO : | de 15 à 30% |
| SrO : | de 0,1 à 10% |
| P₂O₅ : | de 0 à 10% |
| Na₂O : | de 0 à 20% |
| MgO : | de 0 à 10% |
| ZnO : | de 0 à 10% |
| CaF₂ : | de 0 à 5% |
| B₂O₃ : | de 0 à 10% |
| Ag₂O : | de 0 à 10% |
| Al₂O₃ : | de 0 à 3% |
| MnO : | de 0 à 10% |
| Autres : | de 0 à 10% |
les pourcentages étant des pourcentages en masse par rapport à la masse totale du matériau.

2. Matériau selon la revendication 1, **caractérisé en ce que** la somme des masses des éléments SiO₂, CaO, SrO, P₂O₅ représente 98 à 100 % de la masse totale de la composition du matériau de l'invention.

3. Matériau selon la revendication 1 ou la revendication 2, **caractérisé en ce que** sa composition est la suivante :
| | |
|---|---|
| SiO₂ : | de 45 à 75% |
| CaO : | de 15 à 30% |
| SrO : | de 2 à 8% |
| P₂O₅ : | de 0 à 10% |
| Autres éléments : | de 0 à 1% |
en masse par rapport à la masse totale de la composition.

4. Matériau selon l'une quelconque des revendications 1 à 3, **caractérisé en ce qu'**il est sous forme d'une poudre libre d'une poudre compactée, sous forme de fibres, sous forme d'un monolithe ou d'un verre fritté.

5. Matériau selon l'une quelconque des revendications 1 à 3, **caractérisé en ce qu'**il est sous forme d'un revêtement sur un support.

6. Matériau selon la revendication 4, **caractérisé en ce qu'**il est sous forme de poudre et qu'il présente des pores d'une taille comprise entre 1 nm et 50 µm.

7. Verre bioactif **caractérisé en ce qu'**il est issu d'un procédé de fusion et que sa composition se **caractérise par** la présence des éléments ci-dessous dans les proportions indiquées :
| | |
|---|---|
| SiO₂ : | de 45 à 55% |
| Na₂O : | de 10 à 25% |
| CaO : | de 10 à 25% |
| SrO : | de 0,1 % à 10% |
| P₂O₅ : | de 0 à 10% |
| MgO : | de 0 à 10% |
| ZnO : | de 0 à 10% |
| CaF₂ : | de 0 à 5% |
| B₂O₃ : | de 0 à 10% |
| Ag₂O : | de 0 à 10% |
| Al₂O₃ : | de 0 à 3 % |
| MnO : | de 0 à 10% |
| Autres : | de 0 à 10% |
les pourcentages étant des pourcentages en masse par rapport à la masse totale du matériau.

8. Matériau selon la revendication 7, **caractérisé en ce que** la somme des éléments SiO₂, Na₂O, CaO, SrO, P₂O₅ représente 98 à 100 % de la masse totale du matériau de l'invention.

9. Matériau selon la revendication 8, **caractérisé en ce qu'**il est sous forme d'un monolithe ou d'un verre fritté.

10. Composition thérapeutique, comprenant au moins un matériau selon l'une quelconque des revendications 1 à 7 et un support pharmaceutiquement acceptable.

11. Prothèse ou matrice osseuse recouverte partiellement ou sur la totalité de sa surface par un matériau selon l'une quelconque des revendications 1 à 7.

12. Solution bioactive et biocompatible obtenue à partir d'un matériau selon l'une quelconque des revendications 1 à 9, par dissolution du matériau dans un milieu aqueux.

13. Utilisation d'un matériau selon l'une quelconque des revendications 1 à 9, pour la fabrication d'une prothèse ou d'un médicament destiné au comblement d'un défaut osseux.

14. Utilisation d'un matériau selon l'une quelconque des revendications 1 à 9, pour la fabrication d'une prothèse ou d'un médicament destiné à la stimulation de la croissance osseuse.

15. Utilisation d'un matériau selon l'une quelconque des revendications 1 à 9, pour la fabrication d'une prothèse ou d'un médicament destiné à favoriser la réparation et/ou la régénération du cartilage.

## Claims

1. Bioactive glass, **characterised in that** it is made by a sol-gel process and its composition is **characterised by** the presence of the elements below in the specified proportions:
| | |
|---|---|
| SiO₂ : | from 40 to 75% |
| CaO: | from 15 to 30% |
| SrO | from 0.1 to 10% |
| P₂O₅: | from 0 to 10% |
| Na₂O: | from 0 to 20% |
| MgO: | from 0 to 10% |
| ZnO: | from 0 to 10% |
| CaF₂: | from 0 to 5% |
| B₂O₃: | from 0 to 10% |
| Ag₂O: | from 0 to 10% |
| Al₂O₃: | from 0 to 3% |
| MnO: | from 0 to 10% |
| Others: | from 0 to 10%, |
the percentages being percentages by weight relative to the total weight of the material.

2. Material as claimed in claim 1, **characterised in that** the sum of the weights of the elements SiO₂, CaO, SrO, P₂O₅ represents 98 to 100% of the total weight of the composition of the material of the invention.

3. Material as claimed in claim 1 or claim 2, **characterised in that** its composition is as follows:
| | |
|---|---|
| SiO₂: | from 45 to 75% |
| CaO: | from 15 to 30% |
| SrO | from 2 to 8% |
| P₂O₅: | from 0 to 10% |
| Other elements: | from 0 to 1% |
by weight relative to the total weight of the composition.

4. Material as claimed in any one of claims 1 to 3, **characterised in that** it is in the form of a loose powder, compacted powder, in the form of fibres, in the form of a monolith or sintered glass.

5. Material as claimed in any one of claims 1 to 3, **characterised in that** it is in the form of a coating on a substrate.

6. Material as claimed in claim 4, **characterised in that** it is in the form of powder and has pores of a size ranging between 1 nm and 50 µm.

7. Bioactive glass, **characterised in that** it is made by a fusion process and its composition is **characterised by** the presence of the elements below in the specified proportions:
| | |
|---|---|
| SiO₂: | from 45 to 55% |
| Na₂O: | from 10 to 25% |
| CaO: | from 10 to 25% |
| SrO | from 0.1% to 10% |
| P₂O₅: | from 0 to 10% |
| MgO: | from 0 to 10% |
| ZnO: | from 0 to 10% |
| CaF₂: | from 0 to 5% |
| B₂O₃: | from 0 to 10% |
| Ag₂O: | from 0 to 10% |
| Al₂O₃: | from 0 to 3% |
| MnO: | from 0 to 10% |
| Others: | from 0 to 10%, |
the percentages being percentages by weight relative to the total weight of the material.

8. Material as claimed in claim 7, **characterised in that** the sum of the elements SiO₂, Na₂O, CaO, SrO, P₂O₅ represents 98 to 100% of the total weight of the material of the invention.

9. Material as claimed in claim 8, **characterised in that** it is in the form of a monolith or a sintered glass.

10. Therapeutic composition comprising at least one material as claimed in any one of claims 1 to 7 and a pharmaceutically acceptable carrier.

11. Prosthesis or bone matrix coated partially or on the entirety of its surface with a material as claimed in any one of claims 1 to 7.

12. Biocompatible and bioactive solution obtained from a material as claimed in any one of claims 1 to 9 by dissolving the material in an aqueous medium.

13. Use of a material as claimed in any one of claims 1 to 9 for the manufacture of a prosthesis or a medicament intended to fill a bone defect.

14. Use of a material as claimed in any one of claims 1 to 9 for the manufacture of a prosthesis or a medicament intended to stimulate bone growth.

15. Use of a material as claimed in any one of claims 1 to 9 for the manufacture of a prosthesis or a medicament intended to promote the repair and/or regeneration of cartilage.

## Patentansprüche

1. Bioaktives Glas, **dadurch gekennzeichnet, dass** es aus einem Sol-Gel-Verfahren hervorgeht und seine Zusammensetzung durch das Vorhandensein der nachfolgenden Bestandteile in den angegebenen Anteilen gekennzeichnet ist:
| | |
|---|---|
| SiO₂: | 40 bis 75 % |
| CaO: | 15 bis 30 % |
| SrO: | 0,1 bis 10 % |
| P₂O₅: | 0 bis 10 % |
| Na₂O: | 0 bis 20 % |
| MgO: | 0 bis 10 % |
| ZnO: | 0 bis 10 % |
| CaF₂: | 0 bis 5 % |
| B₂O₃: | 0 bis 10 % |
| Ag₂O: | 0 bis 10 % |
| Al₂O₃: | 0 bis 3 % |
| MnO: | 0 bis 10 % |
| Weitere: | 0 bis 10 %, |
wobei die prozentualen Anteile prozentuale Massenanteile in Bezug auf die Gesamtmasse des Materials sind.

2. Material nach Anspruch 1, **dadurch gekennzeichnet, dass** die Summe der Massen der Bestandteile SiO_{2,} CaO, SrO, P₂O₅ 98 bis 100 % der Gesamtmasse der Zusammensetzung des erfindungsgemäßen Materials darstellt.

3. Material nach Anspruch 1 oder Anspruch 2, **dadurch gekennzeichnet, dass** es folgende Zusammensetzung aufweist:
| | |
|---|---|
| SiO₂: | 45 bis 75 Massenprozent |
| CaO: | 15 bis 30 Massenprozent |
| SrO: | 2 bis 8 Massenprozent |
| P₂O₅: | 0 bis 10 Massenprozent |
| Weitere: | 0 bis 1 Massenprozent |
in Bezug auf die Gesamtmasse der Zusammensetzung.

4. Material nach einem der Ansprüche 1 bis 3, **dadurch gekennzeichnet, dass** es in Form eines losen Pulvers, eines kompaktierten Pulvers, in Form von Fasern, in monolithischer Form oder in Form eines Sinterglases vorliegt.

5. Material nach einem der Ansprüche 1 bis 3, **dadurch gekennzeichnet, dass** es in Form eines Überzugs auf einem Träger vorliegt.

6. Material nach Anspruch 4, **dadurch gekennzeichnet, dass** es in Pulverform vorliegt und Poren mit einer Größe zwischen 1 nm und 50 µm aufweist.

7. Bioaktives Glas, **dadurch gekennzeichnet, dass** es aus einem Schmelzverfahren hervorgeht und seine Zusammensetzung durch das Vorhandensein der nachfolgenden Bestandteile in den angegebenen Anteilen gekennzeichnet ist:
| | |
|---|---|
| SiO₂: | 45 bis 55 % |
| Na₂O: | 10 bits 25 % |
| CaO: | 10 bis 25 % |
| SrO: | 0,1 bis 10 % |
| P₂O₅: | 0 bis 10 % |
| MgO: | 0 bis 10 % |
| ZnO: | 0 bis 10 % |
| CaF₂: | 0 bis 5 % |
| B₂O₃: | 0 bis 10 % |
| Ag₂O: | 0 bis 10 % |
| Al₂O₃: | 0 bis 3 % |
| MnO: | 0 bis 10 % |
| Weitere: | 0 bis 10 %, |
wobei die prozentualen Anteile prozentuale Massenanteile in Bezug auf die Gesamtmasse des Materials sind.

8. Material nach Anspruch 7, **dadurch gekennzeichnet, dass** die Summe der Elemente SiO₂, Na₂O, CaO, SrO, P₂O₅ 98 bis 100 % der Gesamtmasse des erfindungsgemäßen Materials darstellt.

9. Material nach Anspruch 8, **dadurch gekennzeichnet, dass** es in monolithischer Form oder in Form eines Sinterglases vorliegt.

10. Therapeutische Zusammensetzung, die mindestens ein Material nach einem der Ansprüche 1 bis 7 und einen pharmazeutisch akzeptablen Träger umfasst.

11. Prothese oder Knochenmatrix, die teilweise oder auf ihrer gesamten Oberfläche mit einem Material nach einem der Ansprüche 1 bis 7 bedeckt ist.

12. Bioaktive und biokompatible Lösung, die ausgehend von einem Material nach einem der Ansprüche 1 bis 9 durch Auflösung des Materials in einem wässrigen Medium erhalten wird.

13. Verwendung eines Materials nach einem der Ansprüche 1 bis 9 zur Herstellung einer Prothese oder eines Arzneimittels, die bzw. das zum Ausgleichen eines Knochendefekts vorgesehen ist.

14. Verwendung eines Materials nach einem der Ansprüche 1 bis 9 zur Herstellung einer Prothese oder eines Arzneimittels, die bzw. das zur Stimulierung des Knochenwachstums vorgesehen ist.

15. Verwendung eines Materials nach einem der Ansprüche 1 bis 9 zur Herstellung einer Prothese oder eines Arzneimittels, die bzw. das zur Unterstützung der Wiederherstellung und/oder Regenerierung von Knorpel vorgesehen ist.
